# EUROPÄISCHE PATENTSCHRIFT

(11) **EP 3 651 754 B1**
(45) Veröffentlichungstag und Bekanntmachung des Hinweises auf die Patenterteilung: **30.04.2025**
(21) Anmeldenummer: 18750103.6
(22) Anmeldetag: 13.07.2018
(51) Int. Cl.: A61K 31/19, A61K 31/198, A23L 33/12, A61P 25/16

(54) **PROPION- UND/ODER BUTTERSÄURE ZUR VERWENDUNG ZUR PROPHYLAKTISCHEN UND/ODER UNTERSTÜTZENDEN THERAPEUTISCHEN BEHANDLUNG VON MORBUS PARKINSON**
PROPIONIC- AND/OR BUTYRIC ACID FOR THE USE FOR THE PROPHYLACTIC AND/OR SUPPORTIVE THERAPEUTIC TREATMENT OF MORBUS PARKINSON
ACIDE PROPIONIQUE ET /OU BUTYRIQUE POUR L'UTILISATION POUR LE TRAITEMENT PROPHYLACTIQUE ET/OU THERAPEUTIQUE ASSISTANTE DANS MORBUS PARKINSON

(30) Priorität: 13.07.2017 DE 102017115819
(43) Veröffentlichungstag der Anmeldung: 20.05.2020
(73) Patentinhaber: Przuntek, Horst, 44801 Bochum (DE); Haghikia, Aiden, 44789 Bochum (DE)
(72) Erfinder: Przuntek, Horst, 44801 Bochum (DE); Haghikia, Aiden, 44789 Bochum (DE)
(74) Vertreter: Schneiders & Behrendt Bochum
(86) Internationale Anmeldenummer: PCT/EP2018/069089
(87) Internationale Veröffentlichungsnummer: WO 2019/012108

(56) Entgegenhaltungen:
- WO-A1-2017/067681
- DE-A1- 19 503 598
- FR-A1- 2 782 608
- US-A1- 2006 105 962
- LORI DAJOSE, SARKIS MAZMANIAN: "Parkinson's Diesase Linked to Microbiome", 12 January 2016 (2016-01-12), XP002785133, Retrieved from the Internet <URL:http://www.caltech.edu/news/parkinsons-disease-linked-microbiome-53109> [retrieved on 20180921]
- UNGER MARCUS M ET AL: "Short chain fatty acids and gut microbiota differ between patients with Parkinson's disease and age-matched controls", PARKINSONISM & RELATED DISORDERS, UK, vol. 32, 31 October 2016 (2016-10-31), pages 66 - 72, XP009508122, ISSN: 1873-5126, DOI: 10.1016/J.PARKRELDIS.2016.08.019
- BOURASSA MEGAN W ET AL: "Butyrate, neuroepigenetics and the gut microbiome: Can a high fiber diet improve brain health?", NEUROSCIENCE LETTERS, ELSEVIER, AMSTERDAM, NL, vol. 625, 8 February 2016 (2016-02-08), pages 56 - 63, XP029593634, ISSN: 0304-3940, DOI: 10.1016/J.NEULET.2016.02.009

## Beschreibung

Die Erfindung betrifft ein Mittel zur prophylaktischen und/oder unterstützenden therapeutischen Behandlung von Morbus Parkinson.

Zahlreiche Erkrankungen gehen einher mit einer Fehlbesiedelung des Darms, d. h. das Mikrobiom des Darms ist aus dem Gleichgewicht geraten, sei es durch eine Fehlbesiedelung oder den Verlust von für den Erhalt von Körperfunktionen wesentlichen Bestandteilen. Bei diesen Erkrankungen, etwa Übergewicht, Colitis Ulcerosa, Multiple Sklerose (MS), Morbus Parkinson und vermutlich auch Psychosen, aber auch beim rheumatischen Formkreis und Psoriasis lassen sich signifikante Veränderungen im Mikrobiom des Darms nachweisen. Dies legt nahe, dass Mikrobiom und Erkrankung miteinander verflochten sind, sei es durch eine gemeinsame Ursache, sei es durch eine wechselseitige Beeinflussung.

Es hat sich ferner gezeigt, dass das Mikrobiom des Darms durch die Art der Nahrung beeinflusst werden kann und in der Lage ist, sich an die Anforderungen der jeweiligen Nahrung anzupassen. Dies bedeutet, dass eine für den Immunstatus für den Patienten ungünstige Darmflora durch diätetische Maßnahmen dahingehend geändert werden kann, dass sich der Immunstatus des Patienten verbessert.

Der Erfindung liegt die Erkenntnis zugrunde, dass bei Morbus Parkinson eine charakteristische Veränderung des Mikrobioms im Vergleich zu gesunden Kontrollpersonen vorliegt. Insbesondere sind im Mikrobiom auch kurzkettige Fettsäuren produzierende Bakterien kaum oder nicht vorhanden. Entsprechend herrscht ein Mangel an kurzkettigen Fettsäuren, die im Darm einer gesunden Kontrollperson als Abbauprodukte auftreten. Braak hat postuliert, dass der Morbus Parkinson im Darm beginnt. Dies steht in Einklang mit dem Befund, dass der Transfer des Mikrobioms von parkinsonkranken Mäusen auf gesunde Mäuse ein Morbus Parkinson vergleichbares Bild hervorruft.

Der bei Parkinsonkranken festgestellte Mangel an kurzkettige Fettsäuren produzierenden Mikroorganismen bewirkt einen Mangel vor allem an Essig-, Propion- und Buttersäure. Während Essigsäure reichlich mit der Nahrung zugeführt wird, wird der Mangel an Propion- und Buttersäure in der Regel nicht ausgeglichen.

Alpha-Synuklein, ein Transportprotein, das im Gehirn auftritt und bei Morbus Parkinson eine Rolle spielt, kann in sehr frühen Krankheitsstadien im Darm nachgewiesen werden und als Indikator für die Krankheit dienen. Auch dies legt einen Zusammenhang zwischen der Erkrankung und dem Geschehen im Darm nahe.

Es sind Versuche bekannt geworden, Defizite in der Darmbesiedlung medikamentös oder durch diätetische Maßnahmen auszugleichen, was aber nur begrenzt erfolgreich war.

M Unger et al., Parkinsonism and Related Disorders 32 (2016), 66-72 diskutiert die abweichende Häufigkeit von Darmbakterien und kurzkettigen Fettsäuren bei Parkinson-Patienten. Im einzelnen wird die Schutzwirkung von Natriumbutyrat auf dopaminerge Neuronen anhand eines Drosophila-Modells besprochen.

S. Sharma et al., XP009544J83 (Sept. 2015) diskutiert die positive Wirkung von Natriumbutyrat auf motorische Defizite bei Parkinson-Ratten, die mit 6-Hydroxydopamin ausgelöst und danach mit Apomorphin behandelt wurden.

Überraschend hat sich gezeigt, dass Propionsäure und Buttersäure eine positive Wirkung auf die Entstehung und den Krankheitsverlauf von Morbus Parkinson haben. Dies gilt auch für deren physiologisch vertretbaren Salze und Ester. Es wurde ferner gefunden, dass die gezielte Verabreichung dieser Stoffe die medikamentöse Behandlung von Morbus Parkinson verbessert, also einen Verstärkungseffekt hat. Insbesondere kann die Dosierung der dopaminergen Medikamente, die üblicherweise zur Behandlung von Morbus Parkinson eingesetzt werden, deutlich vermindert werden.

Entsprechend betrifft die Erfindung ein Mittel zur Verwendung bei der prophylaktischen und/oder unterstützenden therapeutischen Behandlung von Morbus Parkinson, enthaltend eine physiologisch wirksame Menge an Propionsäure und Buttersäure und/oder deren physiologisch vertretbaren Salzen oder Estern.

Das erfindungsgemäße Mittel kann sowohl zu prophylaktischen als auch zu therapeutischen Zwecken an Personen, die eine Disposition zur Parkinsonerkrankung haben, oder an Parkinson-Patienten verabreicht werden. Insbesondere ist das Mittel aber zur unterstützenden therapeutischen Behandlung von Parkinson-Patienten geeignet, die im Übrigen eine konventionelle medikamentöse Behandlung erfahren.

Das erfindungsgemäße Mittel enthält Propionsäure und Buttersäure jeweils allein oder in Kombination als solche enthalten. Bevorzugt ist aber die Verabreichung in Form der physiologisch vertretbaren Salze, wobei die Salze physiologisch wichtiger Metalle im Vordergrund stehen. Dies können neben Alkali- und Erdalkalisalzen insbesondere auch Zink- und Eisensalze sein.

Besonders bevorzugt sind die Natrium-, Kalium-, Magnesium- und Kalziumsalze sowohl der Propionsäure als auch der Buttersäure.

Daneben können Propion- und Buttersäure auch in Form ihrer Ester verabreicht werden. Hier kommen insbesondere die Ester von C₁- bis C₆-Alkoholen in Frage, insbesondere die Methyl- und Ethylester. Die Ester werden im Körper zu freien Säuren hydrolysiert.

Das erfindungsgemäße Mittel kann in üblichen Formen verabreicht werden, beispielsweise in Form von Tabletten, Dragees, Pillen, Kapseln, Pastillen, Pulvern und Granulaten. Eine Verabreichung in flüssiger Form ist ebenfalls möglich in Form von Säften, Tropfen und Tees. In jedem Fall ist das Mittel zur oralen Aufnahme bestimmt.

Bevorzugte Verabreichungsformen sind Tabletten, Kapseln und Pulver. Die Tabletten und Kapseln mit einer Einheitsdosis des erfindungsgemäßen Mittels werden vorzugsweise zweimal täglich verabreicht. Das Pulver kann beispielsweise in ein Getränk eingerührt werden oder aber auch der Nahrung zugemischt werden.

Eine Einheitsdosis für die oben genannten Verabreichungsformen liegt im Bereich von 0,2 bis 5 g, insbesondere 0,3 bis 3 g. Eine besonders bevorzugte Menge für Tabletten, Kapseln und Pulver sind 0,5 bis 2,0 g, jeweils zur morgendlichen und abendlichen Verabreichung bestimmt, gegebenenfalls auch zusätzlich mittäglich, insbesondere im Zusammenhang mit den Mahlzeiten.

Besonders bevorzugt ist die kombinierte Verabreichung von Propion- und Buttersäure oder deren Salzen und Estern in einer Einzeldosis, aber auch in getrennter Form. Dabei kann das Gewichtsverhältnis beispielsweise im Bereich von 3:1 bis 1:3, insbesondere 3:2 bis 2:3, liegen, bei den oben angegebenen Gesamtdosen.

Wie schon erwähnt, kann das erfindungsgemäße Mittel zur unterstützenden therapeutischen Behandlung von Parkinsonkranken eingesetzt werden. In diesem Fall wird es zusätzlich zur üblichen medikamentösen Behandlung gegeben, beispielsweise zusammen mit Levodopa und anderen dopaminergen Medikamenten, wie sie üblicherweise eingesetzt werden. Die Dosierung des erfindungsgemäßen Mittels ist wie oben angegeben.

Die Wirkung von Buttersäure/Butyrat wurde in Patientenstudien, an denen inzwischen mehr als 1.000 Probanden über jeweils wenigstens ein Jahr teilgenommen haben, untersucht. Dabei wurden insgesamt 6 g Buttersäure oder Butyrat (als Salz) zusammen mit der ärztlich verordneten Medikation (Levodopa u. a.) in drei täglichen Dosen morgens, mittags und abends zu den Mahlzeiten verabreicht. Es wurden deutliche Verbesserungen des Allgemeinzustandes, insbesondere bei der Motorik, festgestellt.

Überraschend zeigte sich, dass sich der Dopaminbedarf der mit Buttersäure/Butyrat behandelten Parkinsonpatienten um wenigstens 50 % vermindert hatte, teilweise um bis zu 90 %. Dies ist vor allem deshalb von Bedeutung, weil die bei Parkinsonkranken übliche Medikation nicht nur mit Levodopa zu teilweise erheblichen Nebenwirkungen führt, wie Schwindel, Übelkeit, Tachyarrythmie, Psychosen, Dyskinesien, Kreislaufproblemen.

## Patentansprüche

1. Mittel zur Verwendung bei der prophylaktischen und/oder unterstützenden therapeutischen Behandlung von Morbus Parkinson, enthaltend eine physiologisch wirksame Menge an Propionsäure und Buttersäure und/oder deren physiologisch vertretbaren Salzen oder Estern.

2. Mittel zur Verwendung nach Anspruch 1, **dadurch gekennzeichnet, dass** die Salze der Propionsäure und Buttersäure die Alkali- oder Erdalkalisalze sind.

3. Mittel zur Verwendung nach Anspruch 2, **dadurch gekennzeichnet, dass** die Salze Natrium-, Kalium-, Magnesium- oder Kalziumsalze sind.

4. Mittel zur Verwendung nach einem der vorstehenden Ansprüche, **dadurch gekennzeichnet, dass** die Ester die Methyl- oder Ethylester sind.

5. Mittel zur Verwendung nach einem der vorstehenden Ansprüche in Tabletten-Kapsel- oder Pulverform.

6. Mittel zur Verwendung nach einem der vorstehenden Ansprüche, **dadurch gekennzeichnet, dass** es in Einzeldosen von 0,2 bis 5 g konfektioniert ist.

7. Mittel zur Verwendung nach Anspruch 6, **dadurch gekennzeichnet, dass** eine Einzeldosis 0,5 bis 2,0 g Wirkstoff enthält, gegebenenfalls zusammen mit üblichen Konfektionierungsmitteln und Hilfsstoffen.

8. Mittel zur Verwendung nach einem der vorstehenden Ansprüche, enthaltend Propionsäure und Buttersäure oder deren Salze oder Ester in einem Gewichtsverhältnis von 25/75 bis 75/25.

## Claims

1. Agent for use in the prophylactic and/or supportive therapeutic treatment of Morbus Parkinson, containing a physiologically effective amount of propionic acid and butyric acid and/or physiologically acceptable salts or esters thereof.

2. Agent for use according to claim 1, **characterized in that** the salts of propionic acid and butyric acid are the alkali or alkaline earth salts.

3. Agent for use according to claim 2, **characterized in that** the salts are sodium salts, potassium salts, magnesium salts or calcium salts.

4. Agents for use according to any one of the preceding claims, **characterized in that** the esters are the methyl esters or ethyl esters.

5. Agent for use according to any one of the preceding claims in tablet form, capsule form or powder form.

6. Agent for use according to any one of the preceding claims, **characterized in that** it is assembled in individual doses of 0.2 to 5 g.

7. Agent for use according to claim 6, **characterized in that** a single dose contains 0.5 to 2.0 g of active ingredient, optionally together with conventional assembling agents and excipients.

8. Agent for use according to any one of the preceding claims, containing propionic acid and butyric acid or salts or esters thereof in a weight ratio of 25/75 to 75/25.

## Revendications

1. Agent pour utilisation dans le traitement prophylactique et/ou thérapeutique de soutien de Morbus Parkinson, contenant une quantité physiologiquement efficace d'acide propionique et d'acide butyrique et/ou de leurs sels ou esters physiologiquement acceptables.

2. Agent pour utilisation la revendication 1, **caractérisé en ce que** les sels de l'acide propionique et de l'acide butyrique sont les sels alcalins ou alcalino-terreux.

3. Agent pour utilisation selon la revendication 2, **caractérisé en ce que** les sels sont des sels de sodium, de potassium, de magnésium ou de calcium.

4. Agent pour utilisation selon l'une des revendications précédentes, **caractérisé en ce que** les esters sont les esters méthyliques ou éthyliques.

5. Agent pour utilisation selon l'une des revendications précédentes sous forme de comprimés, de gélules ou de poudre.

6. Agent pour utilisation selon l'une des revendications précédentes, **caractérisé en ce qu'**il est conditionné en doses unitaires de 0,2 à 5 g.

7. Agent pour utilisation selon la revendication 6, **caractérisé en ce qu'**une dose unitaire contient de 0,5 à 2,0 g de substance actif, éventuellement avec des agents de conditionnement et des excipients usuels.

8. Agent pour utilisation selon l'une des revendications précédentes, contenant de l'acide propionique et de l'acide butyrique ou leurs sels ou esters dans un rapport pondéral de 25/75 à 75/25.
